(19) Europäisches Patentamt
European Patent Office
Office européen des brevets

(11) Veröffentlichungsnummer: **0 238 814 B1**

(12) # EUROPÄISCHE PATENTSCHRIFT

(45) Veröffentlichungstag der Patentschrift: **24.06.92**

(51) Int. Cl.$^5$: **A61K 31/495**

(21) Anmeldenummer: **87101616.8**

(22) Anmeldetag: **06.02.87**

Die Akte enthält technische Angaben, die nach dem Eingang der Anmeldung eingereicht wurden und die nicht in dieser Patentschrift enthalten sind.

(54) **Antibakterielle Medikamente für Fische.**

(30) Priorität: **19.02.86 JP 32718/86**

(43) Veröffentlichungstag der Anmeldung:
**30.09.87 Patentblatt 87/40**

(45) Bekanntmachung des Hinweises auf die Patenterteilung:
**24.06.92 Patentblatt 92/26**

(84) Benannte Vertragsstaaten:
**AT BE DE ES FR GB IT NL SE**

(56) Entgegenhaltungen:
**EP-A- 0 210 513**
**EP-A- 0 219 784**

**ANTIMICROB. AGENTS CHEMOTHER., BAND 27, NR. 6, JUNI 1985, SEITEN 966-967, AMER. SOC. FOR MICROBIOL., US, J.F. Reinhardt et al. "Comparative in vitro activities of selected antimicrobial agents against Edwardsielle tarda"**

(73) Patentinhaber: **BAYER AG**

**W-5090 Leverkusen 1 Bayerwerk(DE)**

(72) Erfinder: **Kitao, Tadatoshi, Prof.**
**1-35-1 Jingu**
**Miyazaki City Saitama(JP)**
Erfinder: **Sekiguchi, Noboru**
**59 Zennoji**
**Sakado City Saitama(JP)**
Erfinder: **Hayami, Toshio**
**2-12-10, Minamiohi**
**Shinagawa-ku Tokyo(JP)**

EP 0 238 814 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Antimicrob. Agents Chemother., Band 27, Nr. 4, April 1985, Seiten 643.645, Am. Soc. for Microbiol. US, J.F.Reinhardt et al.: "Comparative in vitro activities of selected antimicrobial agents against Aeromonas species and Plesiomonas shigelloides"

FORTSCHR. ANTIMIKROB. ANTINEOPLAST. CHEMOTHER., BAND 3, NR. 5, 1984, SEITEN 615-620, H. KNOTHE ET AL. "Die antibakterielle Aktivität von Ciprofloxacin, Ofloxacin und Norfloxacin im Vergleich zu anderen Chemotherpeutika gegenüber enteropathogenen Bakterien"

Recent ADV. Chemother., Proc. Int. Congr. Chemother., 14.1985, Antimicrobial Sect.2, Seiten 1521-1522 D. Felmingham et al.: "In vitro evaluation of two new 4-quinolones: S 25930 and S 25932"

Drugs Exp. Clin. Res., Band 11, Nr. 4, 1985, Seiten 253.357 Bioscience Ediprint Inc. M.D. O'Rare et al.: " The comparative in vitro activity of twelve 4-quinolone antimicrobials against enteric pathogens"

Fortschr. Antimikrob. Antineoplast. Chemother., Band 3, Nr. 5, 1984, Seiten 579-588, H. Grimm: "In-vitro-Aktivitäten und Parallelresistenz von Gyrase-Hemmern"

Veterinary Ouarterly, Band 10, Nr.4, Juli 1988, Seiten 211-216, J.F.M. Nouws et al.: "Pharmacokinetics of ciprofloxacin in carp, African catfish and rainbow trout".

Chem. Abstracts 79, 123y +124y

Chem. Abstracts 83, 152365Z

Chem. Abstracts 84, 26134 F

Chem. Abstracts 85, 87538 h

## Beschreibung

Die vorliegende Erfindung betrifft antibakterielle Medikamente für Fische. Insbesondere betrifft die vorliegende Erfindung antibakterielle Medikamente, die für die Verhütung, Heilung und Behandlung von Infektionskrankheiten bei Fischen, die durch Mikroorganismen oder im Wasser lebende Mikroorganismen verursacht werden, geeignet sind und die als wirksamen Inhaltsstoff die Chinolin-Derivate, deren Salze oder deren Hydrate der allgemeinen Formel

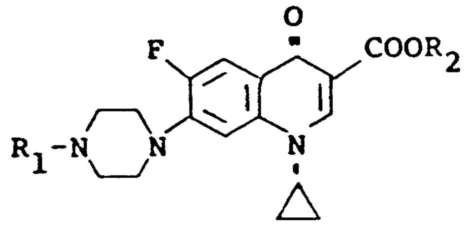

(I)

enthalten, in der

$R_1$ ein Wasserstoff-Atom, eine niedere Alkyl-Gruppe oder eine niedere Hydroxyalkyl-Gruppe bezeichnet und

$R_2$ ein Wasserstoff-Atom oder eine niedere Alkyl-Gruppe bezeichnet.

ausgenommen 1-Cyclopropyl-6-fluor-1,4-dihydro-4-oxo-7-(1-piperazinyl)-chinol on-3-carbonsäure (Ciprofloxacin).

In neuerer Zeit werden wegen des Problems der 200-Meilen-Fischereizone und der durch Überfischen verursachten Verknappung der für den Fang verfügbaren Fischbestände Fischzucht-Kulturen in zunehmend ausgedehntem Maße betrieben. Im Gefolge dieser Tendenz sind Bestrebungen auf eine Hochleistungs-Fischerzeugung gerichtet. Insbesondere in intensiven Fischkulturen mit hoher Belegdichte treten bei den Fischen häufit Infektionskrankheiten auf, und falls solche Krankheiten unbehandelt bleiben, beläuft sich der Schaden gelegentlich auf eine vollständige Zerstörung der Fischkultur in der Zuchtanstalt. Selbst wenn Schaden in diesem Ausmaß nicht auftritt, sind die überlebenden Fische beeinträchtigt und haben einen erheblich verminderten Handelswert. Diese Art Problem tritt vielerorts auf, und es besteht ein starker Bedarf an seiner baldigen und wirksamen Lösung.

Die Mehrzahl der Infektionskrankheiten von Fischen wird durch Mikroorganismen oder im Wasser lebende Mikroorganismen verursacht, und zu ihrer Vorbeugung oder Heilung wurde bisher praktisch so verfahren, daß antibakterielle Medikamente wie Sulfas, Nitrofuran, synthetisiertes Penicillin, Tetracyclin, Makrolid-Antibiotika, Nalidixinsäure, Oxolinsäure, Piromidsäure oder Chloramphenicol zur oralen Verabreichung an die Fische deren Futter zugesetzt werden oder infizierte Fische eine bestimmte Zeitspanne in Wasser, das derartige Antibiotika darin gelöst enthält, ein sogenanntes medizinisches Bad, eingesetzt werden. Die bisher bekannten antibakteriellen Mittel, wie sie oben genannt sind, weisen jedoch die Nachteile auf, daß ihre antimikrobiellen Spektren eng sind, ihre Heilwirkung schwach ist, ihre Sicherheitsspielräume (die Abstände zwischen der wirksamen Dosis und der toxischen Dosis) klein sind, unerwünschte Nebenwirkungen auftreten und ihr Einsatz unwirtschaftlich ist, und aufgrunddessen sind sie nicht recht zufriedenstellend.

Es wurde gefunden, daß die Chinolin-Derivate, deren Salze und deren Hydrate der obigen allgemeinen Formel (I) hervorragende vorbeugende und heilende Wirkungen in breiter Weise gegen verschiedene, durch Mikroorganismen und im Wasser lebende Mikroorganismen hervorgerufene Infektionskrankheiten von Fischen zeigen, daß sie außerdem gegen resistente Bakterien und gegen Komplikationen hochwirksam sind und daß sie geringe Toxizität haben, sicher und rasch metabolisiert werden und sich außerordentlich gut für vorbeugende und heilende antibakterielle Medikamente gegen Infektionskrankheiten von Fischen eignen.

In der obigen Formel (I) umfassen die Niederalkyl-Struktureinheiten in den "niederen Alkyl"- und "niederen Hydroxyalkyl"-Gruppen geradkettige oder kettenverzweigte Alkyl-Gruppen mit bis zu sechs Kohlenstoff-Atomen, vorzugsweise bis zu vier Kohlenstoff-Atomen, z.B. Methyl, Ethyl, n-Propyl, iso-Propyl, n-Butyl, sec-Butyl, iso-Butyl, tert-Butyl etc.. Zu bevorzugten Beispielen für $R_1$ zählen das Wasserstoff-Atom, die Methyl-, die Ethyl- und die $\beta$-Hydroxyethyl-Gruppe. Für $R_2$ werden ebenfalls das Wasserstoff-Atom, die Methyl- und die Ethyl-Gruppe bevorzugt.

Zu typischen Beispielen für die als wirksamer Inhaltsstoff in den antibakteriellen Mitteln der vorliegenden Erfindung verwendbaren Chinolin-Derivate der Formel (I) zählen die folgenden:

Methyl-1-cyclopropyl-6-fluoro-1,4-dihydro-4-oxo-7-piperazino-chinolin-3-carboxylat,

Ethyl-1-cyclopropyl-6-fluoro-1,4-dihydro-4-oxo-7-piperazino-chinolin-3-carboxylat,
1-Cyclopropyl-6-fluoro-1,4-dihydro-4-oxo-7-(4-methylpiperazino)-chinolin-3-carbonsäure,
Ethyl-1-cyclopropyl-6-fluoro-1,4-dihydro-4-oxo-7-(4-methylpiperazino)-chinolin-3-carboxylat,
1-Cyclopropyl-6-fluoro-1,4-dihydro-4-oxo-7-(4-ethylpiperazino)-chinolin-3-carbonsäure,
Ethyl-1-cyclopropyl-6-fluoro-1,4-dihydro-4-oxo-7-(4-ethylpiperazino)-chinolin-3-carboxylat und
1-Cyclopropyl-6-fluoro-1,4-dihydro-4-oxo-7-(4-$\beta$-hydroxyethylpiperazino)-chinolin-3-carbonsäure.

Die Chinolin-Derivate der Formel (I) können unter Ausnutzung der Basizität der Piperazino-Gruppe in der 7-Stellung in der Form von Säureadditionssalzen eingesetzt werden. Beispiele für derartige Säureadditionssalze umfassen Salze mit anorganischen Säuren wie Hydrochloride, Hydrobromide, Hydroiodide und Sulfate und Salze mit organischen Säuren wie Acetate, Citrate, Benzolsulfonate und Embonate (Pamoate). Wenn $R_2$ in den Chinolin-Derivaten der Formel (I) ein Wasserstoff-Atom ist, kann auch die Carboxyl-Gruppe in der 3-Stellung in Salz-Form vorliegen. Zu derartigen Salzen zählen Alkalimetall-Salze wie die Natrium-Salze oder Kalium-Salze, Erdalkalimetall-Salze wie die Magnesium-Salze und Calcium-Salze sowie die Ammonium-Salze, sowie physiologisch verträgliche Salze und Komplexe von Metallen wie Eisen, Kobalt, Kupfer, Mangan, Zink, Aluminium.

Weiterhin können die Chinolin-Derivate der Formel (I) oder deren Salze auch in Form ihrer Hydrate eingesetzt werden.

Die meisten der Chinolin-Derivate der Formel (I) sind an sich bekannte Verbindungen, wie sie beispielsweise in den JP-Offenlegungsschriften 71683/82 und 74667/83 offenbart sind, und können mittels der in diesen Offenlegungsschriften beschriebenen Methoden hergestellt werden.

Zur Verabreichung der Chinolin-Derivate, ihrer Salze oder ihrer Hydrate der Formel (I) [die im Folgenden als die "aktiven Verbindungen der Formel (I)" bezeichnet werden] als antibakterielle Medikamente an Fische können diese zur oralen Verabreichung unter das Fischfutter gemischt werden, oder sie können in dem Wasser gelöst werden, in das man erkrankte Fische einsetzt und in dem man sie herumschwimmen läßt (eine Methode unter Benutzung eines sogenannten "medizinischen Bades").

Außerdem können die aktiven Verbindungen der Formel (I) zu geeigneten Formulierungen für solche Verabreichungsformen wie beispielsweise Pulver, Granulate oder Lösungen als Futter-Zusatzstoffe oder lösliche Dispergierungen oder Lösungen für medizinische Bäder verarbeitet werden. Die Verfahren zur Zubereitung solcher Formulierungen ähneln an sich denjenigen, die normalerweise praktisch bei der Formulierung von Verhütungs- oder Heilmitteln bei akutem Befall angewandt werden. Gemäß allgemeiner Praxis werden einer oder mehrere aus verschiedenen Zusatzstoffen, z.B. Streckmittel wie Sojabohnen-Protein, Lactose, Bierhefe und Kalkstein, Verdünnungsmittel wie Wasser, Lösungsvermittler wie Benzyl-Alkohol, n-Butanol., Eindickungsmittel wie Hydroxypropylmethylcellulose und pH-Regulatoren wie Kaliumhydroxid, Natriumhydroxid, Milchsäure, Salzsäure und Essigsäure in geeigneter Weise mit der aktiven Verbindung vermischt, um die geeignete Formulierungsform herzustellen. Im Folgenden werden die typischen Formen im einzelnen erläutert.

| Formulierungsbeispiel 1 (Pulver als Futter-Zusatz): | |
|---|---|
| Aktive Verbindung der Formel (I) | 1-10 Gew.-Teile |
| Sojabohnen-Protein | 99-90 Gew.-Teile |
| Insgesamt | 100 Gew.-Teile |

Sojabohnen-Protein wird zu der aktiven Verbindung der Formel (I) hinzugefügt, und beide Komponenten werden in einem Mischer homogen miteinander vermischt.

**Formulierungsbeispiel 2 (Lösung als Futter-Zusatz oder für ein medizinisches Bad):**

| | |
|---|---|
| Aktive Verbindung der Formel (I) | 0,5 -10 Gew.-Teile |
| Kaliumhydroxid | 0,08- 1,5 Gew.-Teile |
| Benzylalkohol | 1,3 - 1,4 Gew.-Teile |
| Hydroxypropylmethylcellulose 50 | 0 - 3,5 Gew.-Teile |
| Gereinigtes Wasser | Restmenge |
| Insgesamt | 100 Gew.-Teile |

Kaliumhydroxid wird zu gereinigtem Wasser gegeben, und die Mischung wird gerührt, bis ein homogenes System entstanden ist; zu diesem werden die aktive Verbindung der Formel (I), der Benzylalkohol und die Hydroxypropylmethylcellulose 50 hinzugefügt, und die Mischung wird gerührt, bis sie einheitlich ist.

| Formulierungsbeispiel 3 (Lösliches Pulver für ein medizinisches Bad): | |
|---|---|
| Aktive Verbindung der Formel (I) (eine, die wasserlöslich ist) | 1-10 Gew.-Teile |
| Lactose | 99-90 Gew.-Teile |
| Insgesamt | 100 Gew.-Teile |

Lactose wird zu der aktiven Verbindung der Formel (I) hinzugefügt, und beide Komponenten werden in einem Mischer homogen miteinander vermischt.

**Formulierungsbeispiel 4**

| | |
|---|---|
| Aktive Verbindung der Formel I | 0,1 - 25 Gew.-Teile |
| Kaliumhydroxid | 0,08- 1,5 Gew.-Teile |
| Kaliumchlorid | 0,2 - 2 Gew.-Teile |
| Benzylalkohol | 1,3 - 1,4 Gew.-Teile |
| Glycin | 0 - 2 Gew.-Teile |
| Gereinigtes Wasser | Restmenge |
| | 100 Gew.-Teile |

Kaliumhydroxid und Kaliumchlorid werden in 50 ml Wasser gelöst, die aktive Verbindung , Benzylalkohol, Glycin und die Restmenge Wasser zugegeben und solange gerührt bis die Mischung einheitlich ist.

Beim Einsatz der aktiven Verbindungen der Formel (I) als antibakterielles Medikament für Fische unterscheiden sich die verabreichten Dosierungen je nach dem Zweck der Verabreichung (Verhütung oder Heilung einer Krankheit) sowie dem Typ der Stärke und dem Ausmaß der Infektion der zu behandelnden Fisch-Zielgruppe. Normalerweise kann jedoch eine Dosis im Bereich von 5 bis 1000 mg, vorzugsweise von 20 bis 100 mg, pro 1 kg Körpergewicht der Fische täglich verabreicht werden, entweder auf einmal oder in mehreren Teilmengen zu verschiedenen Zeiten. Es bedarf jedoch keiner besonderen Erläuterung, daß die oben genannte Dosis einen groben Standard darstellt, der je nach dem Alter, Körpergewicht, Krankheitszustand etc. der Fisch-Zielgruppe erniedrigt oder erhöht werden kann. Der Zeitraum der Verabreichung unterliegt keinen besonderen Beschränkungen, jedoch lassen sich innerhalb einer Zeitspanne von 1 bis etwa 10 Tagen normalerweise ausreichende Wirkungen erzielen.

Die durch die vorliegende Erfindung verfügbar gemachten antibakteriellen Medikamente sind breit wirksam gegen verschiedenartige Mikroorganismen oder im Wasser lebende Mikroorganismen, die Infektionskrankheiten bei Fischen auslösen. Beispielsweise zeigen die Medikamente eine potente antibakterielle Wirkung gegen Bakterien, die zu den Gattungen Aeromonas, Edwardsiella, Pasteurella, Pseudomonas, Streptococcus und Vibrio gehören, und sind folglich außerordentlich wirksam zur Vorbeugung, Behandlung und Heilung von Infektionskrankheiten bei gezüchteten Süßwasserfischen und Seefischen wie jungen Gelbschwänzen, Gelbschwänzen, Aalen, Ayus, Forellen, Lachsen, Karpfen etc. sowie bei Aquariumsfischen wie Goldfischen und tropischen Fischen.

Solche ausgezeichneten Aktivitäten der antibakteriellen Mittel der vorliegenden Erfindung werden mit Hilfe der folgenden Tests in vitro und in vivo bewiesen, bei denen die folgenden Verbindungen eingesetzt wurden.

Verbindung A (Stand der Technik gemäß Art.54(3)):

Hydrochlorid von 1-Cyclopropyl-6-fluoro-1,4-dihydro-4-oxo-7-piperazino-chinolin-3-carbonsäure (Zersetzungspunkt: 319-321 °C).

Verbindung B (Stand der Technik gemäß Art.54(3)):

Monohydrat des Hydrochlorids von 1-Cyclopropyl-6-fluoro-1,4-dihydro-4-oxo-7-piperazino-chinolin-3-carbonsäure.

Verbindung C:

Hydrochlorid von 1-Cyclopropyl-6-fluoro-1,4-dihydro-4-oxo-7-(4-methylpiperazino)-chinolin-3-carbonsäure (Zersetzungspunkt: 345-347 °C).

Verbindung D:

1-Cyclopropyl-6-fluoro-1,4-dihydro-4-oxo-7-(4-ethylpiperazino)-chinolin-3-carbonsäure.

Verbindung E:

Dihydrat des Hydrochlorids von 1-Cyclopropyl-6-fluoro-1,4-dihydro-4-oxo-7-(4-ethylpiperazino)-chinolin-3-carbonsäure.

Verbindung F:

Hydrochlorid von 1-Cyclopropyl-6-fluoro-1,4-dihydro-4-oxo-7-(4-β-hydroxyethylpiperazino)-chinolin-3-carbonsäure (Zersetzungspunkt: 327-333 °C).

Verbindung G:

Embonat von 1-Cyclopropyl-6-fluoro-1,4-dihydro-4-oxo-7-(4-ethylpiperazino)-chinolin-3-carbonsäure.

Verbindung H:

Ethyl-1-cyclopropyl-6-fluoro-1,4-dihydro-4-oxo-7-(4-ethylpiperazino)-chinolin-3-carboxylat (Zersetzungspunkt: 187-189 °C).

Verbindung OA (Kontrolle):

1-Ethyl-1,4-dihydro-6,7-methylendioxy-4-oxo-3-chinolin-carbonsäure.

Testbeispiel 1

Antibakterielle Aktivität in vitro gegen pathogene Bakterien für Fische

Jeder der Test-Stämme wurde über Nacht kultiviert, und jede (etwa $10^6$ Zellen/ml enthaltende) Kulturbrühe wurde in Mueller-Hinton-Agar (Difco) inokuliert, dem 1,0 bis 2,0 % Natriumchlorid zugesetzt

worden war und der die Test-Verbindung in den jeweils angegebenen Konzentrationen enthielt. Die minimale, das Wachstum hemmende Konzentration (MIC) wurde für jede Verbindung nach einer Inkubationsperiode von 20 h bei 25-30 °C bestimmt. Die Ergebnisse sind in der folgenden Tabelle 1 angegeben.

## Tabelle 1

**Antibakterielle Aktivität in vitro gegen pathogene Bakterien für Fische (MIC; µg/ml)**

| Test-Bacterium | Verbindung | | | | | | | | Kontrolle |
|---|---|---|---|---|---|---|---|---|---|
| | A | B | C | D | E | F | G | H | OA |
| **Pasteurella piscicida** | | | | | | | | | |
| EH8304 | 0,05 | 0,025 | 0,05 | 0,05 | 0,05 | 0,1 | 0,1 | 25,0 | 0,1 |
| EH8309 | <0,025 | <0,0125 | 0,05 | 0,025 | 0,05 | 0,1 | 0,1 | 25,0 | 0,1 |
| EH8312 | 0,05 | 0,025 | 0,05 | 0,05 | 0,05 | 0,1 | 0,05 | 12,5 | 0,1 |
| EH8313 | 0,05 | 0,025 | 0,05 | 0,05 | 0,05 | 0,1 | 0,05 | 12,5 | 0,1 |
| NC8319 | <0,025 | 0,025 | 0,05 | 0,025 | 0,05 | 0,1 | 0,01 | 25,0 | 0,1 |
| **Vibrio anguillarum** | | | | | | | | | |
| NH8410 | <0,025 | 0,025 | <0,025 | 0,025 | 0,05 | 0,1 | 0,05 | 12,5 | 0,05 |
| NA8340 | <0,025 | 0,025 | <0,025 | 0,025 | 0,05 | 0,1 | 0,05 | 25,0 | 0,05 |
| NA8341 | <0,025 | 0,025 | <0,025 | 0,025 | 0,05 | 0,1 | 0,05 | 25,0 | 0,05 |
| NA8346 | <0,025 | 0,025 | <0,025 | 0,025 | 0,05 | 0,1 | 0,05 | 12,5 | 0,05 |
| NA8347 | <0,025 | 0,025 | <0,025 | 0,025 | 0,05 | 0,05 | 0,05 | 12,5 | 0,05 |
| **Aeromonas hydrophila** | | | | | | | | | |
| KA8301 | <0,025 | <0,0125 | <0,025 | 0,025 | 0,05 | 0,05 | 0,05 | 12,5 | <0,0125 |
| KA8414 | <0,025 | <0,0125 | <0,025 | <0,0125 | <0,025 | <0,025 | 0,05 | 25,0 | <0,0125 |
| KA8416 | <0,025 | <0,0125 | <0,025 | <0,0125 | <0,025 | <0,025 | 0,05 | 12,5 | <0,0125 |
| M 183 | <0,025 | <0,0125 | <0,025 | <0,0125 | <0,025 | <0,025 | 0,05 | 12,5 | <0,0125 |
| C 44 | <0,025 | <0,0125 | <0,025 | <0,0125 | <0,025 | <0,025 | 0,05 | 50,0 | <0,0125 |

### Testbeispiel 2

Antibakterielle Aktivität der Verbindung D in vitro gegen verschiedene pathogene Bakterien für Fische

Jeder der Test-Stämme wurde über Nacht kultiviert, und jede (etwa $10^6$ Zellen/ml enthaltende) Kulturbrühe wurde in Mueller-Hinton-Agar (Difco) inokuliert, dem 1,0 bis 2,0 % Natriumchlorid zugesetzt worden war und der die Test-Verbindung in den jeweils angegebenen Konzentrationen enthielt. Die

minimale, das Wachstum hemmende Konzentration (MIC) wurde für jede Verbindung nach einer Inkubationsperiode von 20 h bei 25-30 °C bestimmt. Die Ergebnisse sind in der folgenden Tabelle 2 angegeben.

Tabelle 2

Antibakterielle Aktivität der Verbindung D in vitro gegen pathogene Bakterien für Fische

| Test-Bacterium | Zahl der Stämme | (MIC; µg/ml) Verbindung D | Oxolinsäure |
|---|---|---|---|
| Aeromonas hydrophila | 25 | <0,0125-0,05 | <0,0125-0,025 |
| Aeromonas salmonicida | 25 | 0,0125-0,1 | 0,05-3,2 |
| Edwardsielle tarda | 25 | 0,025-0,2 | 0,05-1,6 |
| Pasteurella piscicida | 25 | <0,0125-0,05 | 0,025-0,1 |
| Pseudomonas anguilliseptica | 18 | <0,0125-0,025 | <0,0125-0,05 |
| Streptococcus sp. | 25 | 0,8 | 200-400 |
| Vibrio anguillarum | | | |
| Herkunft: Gelbschwanz | 25 | 0,05-0,1 | 0,025-0,05 |
| Herkunft: Ayu | 25 | 0,025-0,4 | 0,05-25 |

Von den in den vorstehenden Testbeispielen verwendeten aktiven Verbindungen wird im Folgenden als Hinweis ein Herstellungsbeispiel für die Verbindung H angegeben.

Herstellungsbeispiel 1

Zu einer Suspension von 18 g 1-Cyclopropyl-7-(4-ethyl-1-piperazinyl)-6-fluoro-1,4-dihydro-4-oxo-chinolin-3-carbonsäure in 120 ml Ethanol wurden 24 g 96-proz. konzentrierte Schwefelsäure tropfenweise hinzugefügt. Die erhaltene Mischung wurde 12 h zum Rückfluß erhitzt, und nach Entfernen des überschüssigen Alkohols unter vermindertem Druck wurde der Rückstand in 200 ml Eiswasser gelöst. Unter Kühlen mit Eis wurde der pH des Systems durch Zugabe einer kalten Lösung von 19,2 g Natriumhydroxid in 100 ml Wasser auf 12 bis 14 gebracht, und der in der Zwischenzeit entstandene Niederschlag wurde unter Absaugen filtriert. Der Niederschlag wurde dann mit Wasser gewaschen und bei 100 °C unter vermindertem Druck getrocknet. Nach dem Umkristallisieren desselben aus Toluol/Leichtbenzin wurden 10 g des Ethylesters der 1-Cyclopropyl-7-(4-ethyl-1-piperazinyl)-6-fluoro-1,4-dihydro-4-oxo-chinolin-3-carbonsäure (52,5 % der theoretischen Menge) erhalten, der bei 187-189 °C schmilzt.

## Patentansprüche

1. Verwendung von Chinolin-Derivaten, deren Salze oder deren Hydrate der allgmeinen Formel I

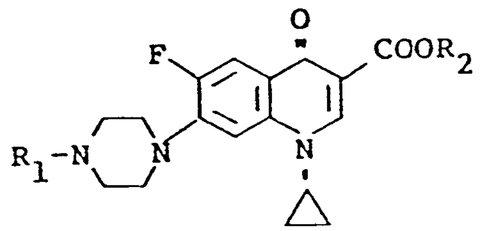

(I)

in der
$R^1$ ein Wasserstoff-Atom, eine niedere Alkyl-Gruppe oder eine niedere Hydroxyalkyl-Gruppe bezeichnet und
$R^2$ ein Wasserstoff-Atom oder eine niedere Alkyl-Gruppe bezeichnet

ausgenommen 1-Cyclopropyl-6-fluor-1,4-dihydro-4-oxo-7-(1-piperazinyl)-chinolon-3-carbonsäure (Ciprofloxacin) zur Herstellung von antibakteriellen Medikamenten für Fische.

**2.** Verwendung gemäß Anspruch 1, dadurch gekennzeichnet, daß als Chinolin-Derivat der Formel I 1-Cyclopropyl-6-fluor-1,4-dihydro-4-oxo-7(4-ethylpiperazin)-chinolin-3-carbonsäure (Enrofloxacin), seine Salze oder Hydrate, eingesetzt wird.

**Claims**

**1.** Use of quinoline derivatives, their salts or their hydrates of the general formula (I)

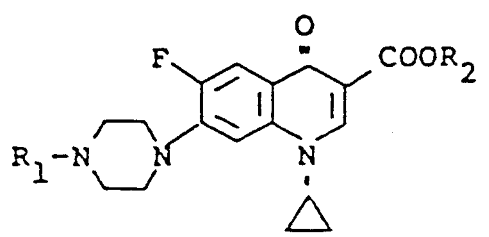

(I)

in which
$R^1$ indicates a hydrogen atom, a lower alkyl group or a lower hydroxyalkyl group and
$R^2$ indicates a hydrogen atom or a lower alkyl group excepting 1-cyclopropyl-6-fluoro-1,4-dihydro-4-oxo-7-(1-piperazinyl)quinoline-3-carboxylic acid (ciprofloxacin) for the preparation of antibacterial medicaments for fish.

**2.** Use according to Claim 1, characterised in that 1-cyclopropyl-6-fluoro-1,4-dihydro-4-oxo-7-(4-ethylpiperazine) quinoline-3-carboxylic acid (enrofloxacin), its salts or hydrates is employed as the quinoline derivative of the formula I.

**Revendications**

**1.** Utilisation de dérivés de quinoléine, de leurs sels ou de leurs hydrates répondant à la formule générale I

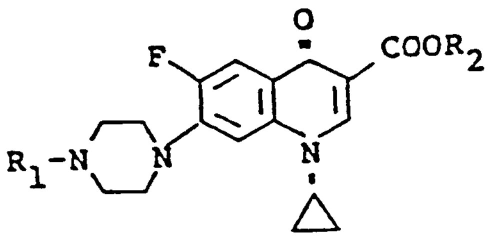

(I)

dans laquelle

R1 représente un atome d'hydrogène, un groupe alkyle inférieur ou un groupe hydroxyalkyle inférieur et

R2 représente un atome d'hydrogène ou un groupe alkyle inférieur,

à l'exclusion de l'acide 1-cyclopropyl-6-fluoro-1,4-dihydro-4-oxo-7-(1-pipérazinyl)-quinolone-3-carboxylique (Ciprofloxacine), pour la préparation de médicaments antibactériens destinés à des poissons.

**2.** Utilisation selon la revendication 1, **caractérisée en ce qu'**on met en oeuvre, comme dérivé de quinoléine de formule I, l'acide 1-cyclopropyl-6-fluoro-1,4-dihydro-4-oxo-7-(4-éthylpipérazino)-quinoléine-3-carboxylique (Enrofloxacine), ses sels ou ses hydrates.